# EUROPEAN PATENT SPECIFICATION

(11) **EP 2 540 210 B1**
(45) Date of publication and mention of the grant of the patent: **27.05.2015**
(21) Application number: 11812533.5
(22) Date of filing: 27.07.2011
(51) Int. Cl.: A61B 1/00

(54) **MEDICAL INSTRUMENT HOLDING DEVICE**
VORRICHTUNG ZUM HALTEN EINES MEDIZINISCHEN INSTRUMENTS
DISPOSITIF DE SUPPORT D'INSTRUMENT MÉDICAL

(30) Priority: 29.07.2010 JP 2010170987
(43) Date of publication of application: 02.01.2013
(73) Proprietor: OLYMPUS MEDICAL SYSTEMS CORP., Tokyo 151-0072 (JP)
(72) Inventor: KANAZAWA, Noriaki, Tokyo 192-8512 (JP); HARANO, Kenji, Tokyo 192-8512 (JP)
(74) Representative: Schicker, Silvia
(86) International application number: PCT/JP2011/067142
(87) International publication number: WO 2012/014944

(56) References cited:
- JP-A- 7 227 398
- JP-A- 10 272 143
- JP-A- 2000 316 873
- JP-A- 2004 337 358
- JP-A- 2005 000 645
- JP-A- 2005 118 457
- JP-A- 2005 192 743
- JP-A- 2005 192 743
- JP-A- 2005 324 038
- JP-A- 2006 218 205
- JP-A- 2006 218 205
- JP-A- 2006 254 974
- JP-A- 2007 152 129
- US-A1- 2004 210 106
- US-A1- 2009 306 469

## Description

### Technical Field

The present invention relates to a supporting apparatus for medical device, which supports an endoscope for observing inside of a body cavity of a subject to inspect, an operation microscope for observing inside of a trepanned head, or a treatment instrument for curative treatment on an affected part.

### Background Art

In surgical operations in neurosurgery, there are employed a method of magnifying and observing a micro surgical site by an operation microscope. As supporting apparatuses for the microscope, there have been developed a supporting apparatus for medical device, which comprises a balance mechanism to maintain a three-dimensional balance owing to the principle of balance, and a supporting apparatus for medical device, which comprises a balance mechanism to maintain a balance in up and down directions owing to reaction force of a spring.

In surgical operations in digestive surgery, there is employed a method of observing inside of a body cavity by a hard mirror. Although the hard mirror is held by a hand in common, surgical operations can require a long time. Therefore, there is difficulty in stably observing an arbitrary portion for a long time with a hard mirror held by a hand. Hence, development is made in a supporting apparatus for medical device, which comprises a balance mechanism to support a hard mirror in a state of balancing a medical microscope.

In endoscope inspections in digestive tract internal medicine, there is employed a method of observing inside of a body cavity by a flexible endoscope. In recent years, advanced curative treatments complicate manipulation of endoscopes and elongate treatment time. Accordingly, it is effective to support even a flexible endoscope by a supporting apparatus for medical device, which comprises a balance mechanism to maintain a balance.

As an example of a balance mechanism comprised in a supporting apparatus for medical device, Jpn. Pat. Appln. KOKAI Publication No. 57-860806 discloses a balance mechanism which maintains a balance in up and down directions owing to reaction force of a spring.

Further, Jpn. Pat. Appln. KOKAI Publications No. 63-36481 and 7-227398 disclose balance mechanisms which maintain a three-dimensional balance owing to the principle of balance.

Specifically, Jpn. Pat. Appln. KOKAI Publication No. 57-860806 discloses a supporting apparatus for medical device, which comprises a parallelogram link mechanism. A balance in up and down directions of an observation apparatus is maintained by providing an adjustable spring within this parallelogram link mechanism.

The supporting apparatus for medical device disclosed in Jpn. Pat. Appln. KOKAI Publication No. 63-36481 is configured to maintain a three-dimensional balance of an optical observation apparatus by providing a pivot fulcrum on a line connecting centroids of the optical observation apparatus and a counter weight. A supporting apparatus for surgical instrument disclosed in Jpn. Pat. Appln. KOKAI Publication No. 7-227398 is configured to fix/release a joint part by an electromagnetic brake built in the joint part, in addition to the configuration of maintaining a three-dimensional balance of an optical observation apparatus by providing a pivot fulcrum on a line connecting centroids of the optical observation apparatus and a counter weight.

In the aforementioned supporting apparatus for medical device disclosed in Jpn. Pat. Appln. KOKAI Publication No. 57-86806, movement operation can be achieved with a balance constantly maintained in up and down directions of the observation apparatus owing to reaction force of a spring, when the observation apparatus is attached to an arm of the supporting apparatus for medical device. Therefore, an operator can easily change an observation view field in the up and down directions of the observation apparatus and can make fine adjustment also in the up and down directions.

However, when the observation apparatus is detached from the arm, a tip end of the parallelogram jumps up owing to reaction force of the spring. Therefore, when replacement is required with another observation apparatus adequate for a surgical method during a surgical operation, labor of repositioning is required after replacement with the observation apparatus because observation apparatuses cannot be replaced maintained at an arbitrary position.

In the aforementioned supporting apparatus for medical device disclosed in Jpn. Pat. Appln. KOKAI Publication No. 63-36481, movement is allowed with a balance constantly maintained in three-dimensional directions of the optical observation apparatus when the optical observation apparatus is attached to an arm of the supporting apparatus for medical device. Therefore, an operator can easily change an observation view field in the three-dimensional directions of the optical observation apparatus and can make fine adjustment also in the three-dimensional directions. However, when the optical observation apparatus is detached from the arm of the supporting apparatus for medical device, a tip end of the supporting apparatus jumps up owing to the weight of a counter weight. Therefore, when replacement is required with another optical observation apparatus adequate for a surgical method during a surgical operation, repositioning to an observation position requires much labor after replacement with the optical observation apparatus because optical observation apparatuses cannot be replaced maintained at arbitrary positions.

In the aforementioned in Jpn. Pat. Appln. KOKAI Publication No. 7-227398, movement operation is allowed with a balance constantly maintained in the three-dimensional directions of a surgical instrument when the surgical instrument is attached to an arm of the supporting apparatus for medical device. Therefore, an operator can change an observation view field in the three-dimensional directions of the surgical instrument, can make fine adjustment also in the three-dimensional directions, and can fix the surgical instrument by an electromagnetic brake built in a joint part through one-touch operation. Further, fixing of the joint part is released only while a brake switch to release fixing of the electromagnetic brake is pressed. Therefore, even when the surgical instrument is detached from the arm, the tip end of the supporting apparatus for medical device does not jump up.

Accordingly, when replacement is made with another surgical instrument adequate for a surgical method during a surgical operation, surgical instruments can be replaced maintained at an arbitrary position. However, when the brake switch is pressed after detaching the surgical instrument, the tip end of the supporting apparatus for surgical instrument jumps up owing to the weight of the counter weight. Therefore, when the brake switch is pressed carelessly, surgical instruments cannot be replaced maintained at an arbitrary position. Therefore, labor of repositioning to an observation position is required after replacement of the surgical instruments.

Japanese patent application published as JP 2006-218205 discloses a support aparatus for surgical apparatus which selectively controls fixing or releasing operation of joints of an arm mechanism according to type of surgery apparatus. This allows the holding of different types of surgical apparatuses in optimal operating positions and highly accurate movement of various surgical apparatuses while having a simple structure.

The invention has an object of providing a supporting apparatus for medical device, which can maintain a balance of a medical device at an arbitrary position and can prevent a tip end of an arm mechanism from jumping up even after detaching the medical device.

### Disclosure of Invention

According to an embodiment of the present invention, there is provided a supporting apparatus according to claim 1.

### Brief Description of Drawings

FIG. 1 is a side view showing a schematic configuration of an entire supporting apparatus for medical device, according to the first embodiment of the invention;
FIG. 2 is a longitudinal sectional view of a main part, showing a fixing release state of a first joint of a link mechanism part owing to a lock part of the supporting apparatus for medical device, according to the first embodiment;
FIG. 3 is a longitudinal sectional view of a main part, showing a fixed state of the first joint by a lock part of the supporting apparatus for medical device, according to the first embodiment;
FIG. 4 is a longitudinal sectional view of a main part, showing a flexible endoscope used in the supporting apparatus for medical device, according to the first embodiment;
FIG. 5 shows a longitudinal sectional view of a main part, showing a fixing release state of releasing fixing of a joint axis of the supporting apparatus for medical device, according to the first embodiment;
FIG. 6 is a longitudinal sectional view of a main part, showing a separate state of a connection part between a drive part and an insertion part of the flexible endoscope, used in the supporting apparatus for medical device;
FIG. 7 is a block diagram showing a connection state of a controller in the supporting apparatus for medical device, according to the first embodiment;
FIG. 8 is a flowchart explaining control of fixing/release of joint parts by the controller in the supporting apparatus for medical device;
FIG. 9 is a schematic diagram of the entire supporting apparatus for medical device, according to the second embodiment of the invention;
FIG. 10 is a longitudinal sectional view of a main part showing a connection part of a flexible endoscope, used in a supporting apparatus for medical device, according to the third embodiment of the invention;
FIG. 11 is a block diagram showing a connection state of a controller in the supporting apparatus for medical device, according to the third embodiment;
FIG. 12 is a flowchart for explaining control of fixing/release of joint parts by the controller of the supporting apparatus for medical device, according to the third embodiment;
FIG. 13 is a plan view showing an operation part of a flexible endoscope used in a supporting apparatus for medical device, according to the fourth embodiment of the invention;
FIG. 14 is a block diagram showing a connection state of a controller in the supporting apparatus for medical device, according to the fourth embodiment;
FIG. 15 is a flowchart explaining control of fixing/release of the joint parts by the controller of the supporting apparatus for medical device, according to the fourth embodiment;
FIG. 16 is a flowchart explaining control of fixing/release of the joint parts by the controller of the supporting apparatus for medical device, according to the fifth embodiment of the invention;
FIG. 17 is a plan view showing an operation part of a flexible endoscope used in a supporting apparatus for medical device, according to the sixth embodiment;
FIG. 18 is a block diagram showing a connection state of a controller in the supporting apparatus for medical device, according to the sixth embodiment; and
FIG. 19 is a flowchart explaining control of fixing/release of joint parts in the supporting apparatus for medical device, according to the sixth embodiment.

### Mode for Carrying Out the Invention

Hereinafter, embodiments of the invention will be described with reference to the drawings.

### First Embodiment

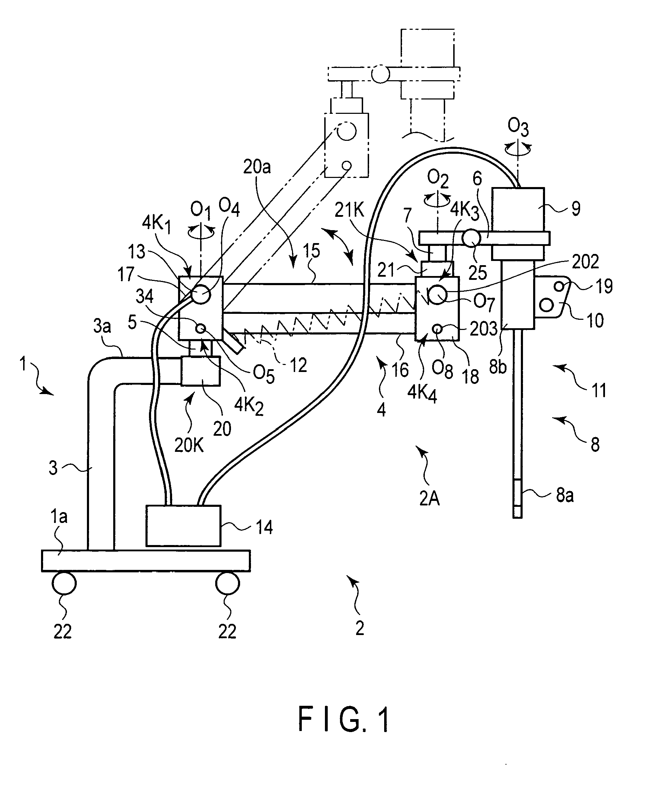

FIGS. 1 to 8 show the first embodiment of the invention. FIG. 1 shows a schematic configuration of an entire supporting apparatus 2 for medical device, according to the present embodiment, which supports a flexible endoscope 11 as a medical device to be movable and tiltable. FIG. 1 shows a stand unit 2 in the supporting apparatus 2 for medical device. To the stand unit 1, a plurality of casters 22 for movement are attached below a base plate 1a as a base. On the base plate 1a, a post 3 is provided to stand in a perpendicular direction. At an upper end part of the post 3, there is provided a horizontal arm part 3a which is bent in a horizontal direction.

At a tip end part of the horizontal arm part 3a, a supporting part 2A of the flexible endoscope 11 is provided. The supporting part 2A comprises a link mechanism part (arm mechanism) 4 made of a parallelogram link which supports the flexible endoscope 11 to be movable and tiltable. The link mechanism part 4 comprises an upper plate 15, a lower plate 16, a rear-link end part 17, and a tip-link end part 18.

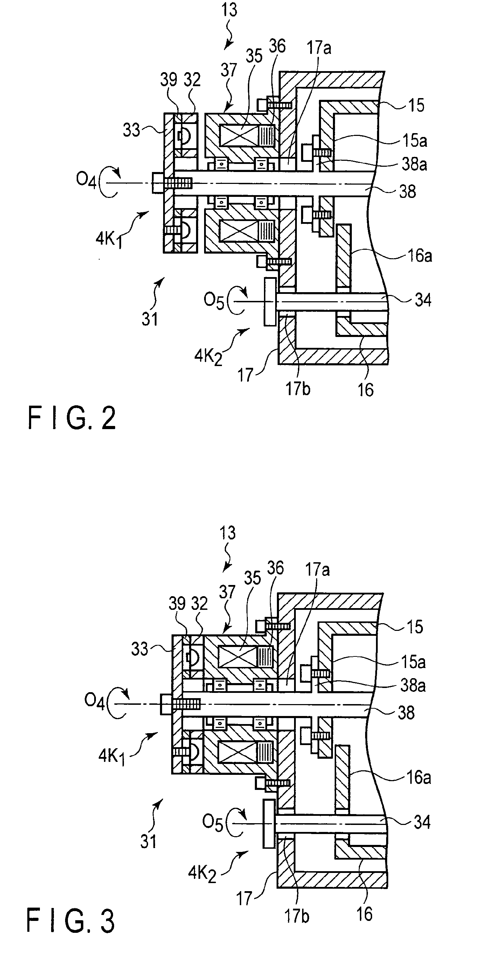

As shown in FIG. 2, the upper plate 15 is a plate-type member having a substantially U-shaped cross-section. A shaft 38 extending in the horizontal directions is inserted in and between two end plates 15a and 15b of the upper plate 15. Two flange parts 38a in contact with the two end plates 15a of the upper end plate 15 are formed on the shaft 38. One of the flange parts 38a is fixed by a bolt to one end plate 15a of the upper plate 15. Similarly, the other one of the flange parts 38a not shown in the figure is also fixed by a bolt to the other one end plate 15a of the upper plate 15. The lower plate 16 is a plate-type member having a substantially U-shaped cross-section. A shaft 34 extended in a horizontal direction is inserted in and between two end plates 15a and 15b of the lower plate 16.

An insertion hole 17a in which the shaft 38 of the upper plate 15 is inserted and an insertion hole 17b in which the shaft 34 of the lower plate 16 is inserted are formed in the rear link end part 17. Further, a base end part of the upper plate 15 (a connection part to the rear link end part 17) is supported to be pivotal about a rotation shaft 04 by the shaft 38 inserted in the insertion hole 17a of the rear link end part 17. Between the upper plate 15 and the rear link end part 17, a first joint 4k1 is formed. Similarly, a base end of the lower plate 16 (connection part to the rear link end 17) is supported to be pivotal about a rotation shaft 05 inserted in the insertion hole 17b of the rear link end part 17 by the shaft 34. Between the lower plate 16 and the rear link end part 17, a second joint 4k2 is formed.

Further, a tip end part of the upper plate 15 (connection part to the rear link end part 18) is supported at the tip-link end part 18, to be pivotal about a rotation axis 07 by a shaft 202. Between the upper plate 15 and the tip-link end part 18, a third joint 4k3 is formed. Similarly, a tip end part of the lower plate 16 (connection part to the tip-link end part 18) is supported at the tip-link end part 18, to be pivotal about a rotation axis 08 by a shaft 203. Between the lower plate 16 and the tip-link end part 18, a fourth joint 4k4 is formed.

Further, by a parallelogram link configured by the upper plate 15, lower plate 16, rear link end part 17, and tip-link end part 18, the link mechanism part 4 is supported to be movable to arbitrary upper and lower positions, in a manner that the parallelogram link deforms to a horizontal position denoted by a continuous line in FIG. 1, an upwardly pivoted position denoted by a virtual line in FIG. 1, and a downwardly pivoted position not shown.

At the tip end part of the horizontal arm part 3a, there are provided a first joint rotation part 20k and a first lock part 20. The first joint rotation part 20k is supported to be pivotal about a rotation axis O1 in a perpendicular direction. The first lock part 20 switches to either a fixed state of fixing movement of the first joint rotation part 20k or a fixing release state of releasing fixing of the first joint rotation part 20k. A support member 5 of the link mechanism part 4 is connected to the first lock part 20. The support member 5 is supported to be pivotal about the rotation axis O1 in the direction perpendicular to the first lock part 20.

Above the tip end of the tip-link end part 18 of the link mechanism part 4, there are provided a second joint rotation part 21k and a second lock part 21. The second joint rotation part 21k is supported to be pivotal about a rotation axis 02 in the perpendicular direction. The second lock part 21 switches to either a fixed state of fixing movement of the second joint rotation part 21k or a release state of releasing fixing of the second joint rotation part 21k.

The support member 7 of the supporting part 6 of the flexible endoscope 11 as a medical device is connected to the second lock part 21. The support member 7 is supported to be pivotal about the rotation axis 02 in the direction perpendicular to the second lock part 21. The supporting part 6 fixes the flexible endoscope 11 as a medical device by a manual brake 25, to be tiltable about the center line of the manual brake 25 as a center.

The flexible endoscope 11 is configured by, for example, an electric bendable endoscope. The electric bendable endoscope comprises an elongate insertion part 8 to be inserted into a body cavity, and a drive part 9 connected to a base end part of the insertion part 8. The drive part 9 is attached or namely fixed to the supporting part 6 of the flexible endoscope 11. The base end part of the insertion part 8 is detachably connected to the drive part 9. Further, the drive part 9 of the flexible endoscope 11 is connected to a tip end of the supporting part 6, to be pivotal about a rotation axis 03.

At a tip end part of the insertion part 8, there is provided a bending part 8a which can be operated, for example, to bend in four directions of up, down, left, and right directions. Further, inside the insertion part 8, there are provided a plurality of bending operation wires (UD wire 8w1 for up and down directions, and RL wire 8w2 in left and right directions).

The drive part 9 is also provided with, for example, two drive motors 9m1 and 9m2, and two drive force transmission mechanisms 9d1 and 9d2. These drive motors 9m1 and 9m2 generate drive forces to draw and drive the bending operation wires 8w1 and 8w2. The drive force transmission mechanisms 9d1 and 9d2 respectively convert drive forces of the drive motors 9m1 and 9m2 into traction forces for the bending operation wires 8w1 and 8w2.

Of these motors, one drive motor 9m1 is a UD motor for driving the bending part 8a to bend in the up and down directions (up (U) direction and down (D) direction), and the other drive motor 9m2 is a RL motor for driving the bending part 8a to bend in the left and right directions (right (R) and left (L) directions).

Further, a connection part 8b which is detachably connected to the drive part 9 is provided at a base end part of the insertion part 8. Clutch parts 8c1, 8c2, 9c1, and 9c2 are provided at a connection part between the connection part 8b of the insertion part 8 and the drive part 9. The clutch parts 8c1, 8c2, 9c1, and 9c2 shut off or connect drive forces which are transmitted between the bending operation wires 8w1 and 8w2 and the drive force transmission mechanisms 9d1 and 9d2.

Further, when the drive part 9 and the connection part 8b of the insertion part 8 are detachably connected to each other, the clutch parts 9c1 and 9c2 of the drive force transmission mechanisms 9d1 and 9d2 and the clutch parts 8c1 and 8c2 of the connection part 8b of the insertion part 8 are separably engaged to connect. In this manner, the drive force from one drive motor 9m1 of the drive part 9 is transmitted from the drive force transmission mechanism 9d1 on a side of the drive part 9 through engaged parts between the clutch parts 9c1 and 8c1. The bending operation wire 8w1 is drawn to operate the bending part 8a to bend in an operating direction of the bending operation wire 8w1. Further, the drive force from the other one drive motor 9m2 of the drive part 9 is transmitted from the drive force transmission mechanism 9d2 on the side of the drive part 9 through engaged parts between the clutch parts 9c2 and 8c2. The bending operation wire 8w1 is drawn to operate the bending part 8a to bend in an operating direction of the bending operation wire 8w2.

The supporting apparatus 2 for medical device, according to the present embodiment, comprises a balance mechanism 201 which maintains a balance of the flexible endoscope 11 attached to the tip end side of the link mechanism part 4, at an arbitrary upper or lower position, owing to reaction force of the compression spring 12. Here, an end of the compression spring 12 is fixed to an upper end part of the tip-link end part 18. The other end of the compression spring 12 is fixed to a lower end part of the rear link end part 17. Owing to the reaction force of the compression spring 12, the weight of the flexible endoscope 11 and the link mechanism part 4 are balanced by the reaction force of the compression spring 12, owing to reaction force of the compression spring 12.

Further, the supporting apparatus 2 for medical device, according to the present embodiment, is provided with a lock part (brake part) 13 which can switch at least one joint part of the link mechanism part 4 at the supporting part 2A of the flexible endoscope 11, i.e., a first joint 4k1 in the present embodiment between a fixed state of fixing the first joint 4k1 and a fixing release state of releasing the fixed first joint 4k1.

The lock part 13 is provided with a non-excitation-operation-type electromagnetic brake 31. This non-excitation-operation-type electromagnetic brake 31 comprises an armature 32 as a movable part and a stator 37 as a stationary part.

The stator 37 is fixed to the rear link end part 17 to be coaxial with a rotation axis 04. The stator 37 of the non-excitation-operation-type electromagnetic brake 31 internally comprises a permanent magnet 35 and a coil 36. Inside the stator 37, the shaft 38 is rotatably supported by a bearing.

A disc-type plate 33 is fixed to one end surface of the shaft 38. The armature 32 is opposed to the plate 33 over a plate spring 39. An end part of the plate spring 39 is fixed to the plate 33, and the other end part thereof is fixed to the armature 32. The armature 32 is fixed to the plate 33 over the plate spring 39.

Further, a suctioned state of the armature 32 due to the permanent magnet 35 is controlled by switching conduction of the stator 37 to the coil 36. When the coil 36 of the stator 37 is not conducted, the armature 32 is maintained in a fixed state in which the armature 32 is suctioned to the stator 37, due to the magnetic force of the permanent magnet 35, as shown in FIG. 3. At this time, the shaft 38 is maintained fixed to the rear link end part 17. Therefore, the shaft 38 in the upper plate 15 is maintained not rotatable, and is thereby maintained in a fixed state of fixing movement of the link mechanism part 4.

When the coil 36 of the stator 37 is switched from a non-conductive state to a conductive state, magnetic force of the permanent magnet 35 is lost, and spring force of the plate spring 39 separates the armature 32 away from the stator 37, as shown in FIG. 2. Therefore, a state is switched into a state in which the shaft 38 is released from being fixed to the rear link end part 17. Therefore, the shaft 38 in the upper plate 15 is switched to be rotatable, and the link mechanism part 4 is switched to a fixing release state.

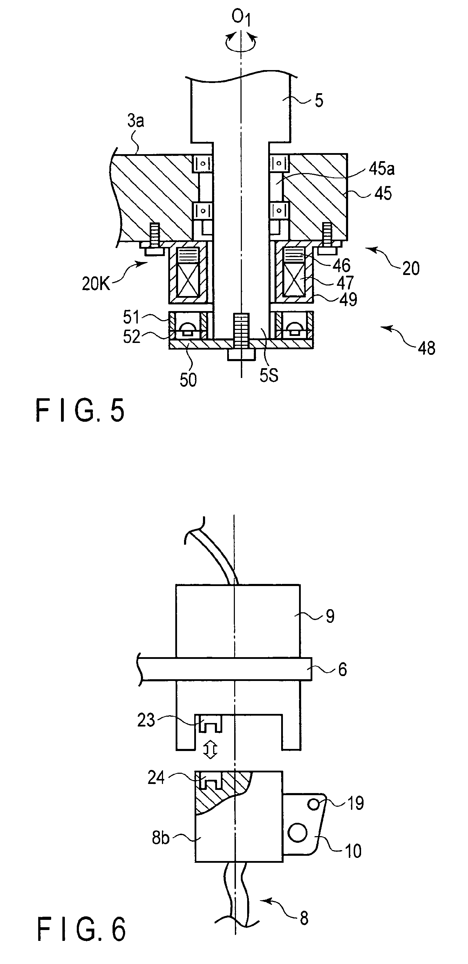

Also, FIG. 5 shows a schematic configuration of the first joint rotation part 20k and the first lock part 20. An insertion hole 45a, in which a shaft 5s at a lower end part of a support member 5 is inserted to be coaxial with the rotation axis O1, is formed in the tip end part 45 of the horizontal arm part 3a of the post 3. The shaft 5s is supported to be rotatable in the insertion hole 45a at the tip end part 45 of the horizontal arm part 3a through a bearing. In this manner, the first joint rotation part 20k which supports the link mechanism part 4 to be pivotal about the rotation axis O1 by the supporting member 5.

The first lock part 20 is provided with a non-excitation-operation-type electromagnetic brake 48 which has the same configuration as the lock part 13 of the first joint 4k1. This non-excitation-operation-type electromagnetic brake 48 comprises a stator 49 as a stationary part and an armature 51 as a movable part. The stator 49 is fixed to the tip end part 45 to be coaxial with the rotation axis 04. The stator 49 of the non-excitation-operation-type electromagnetic brake 48 internally comprises a permanent magnet 47 and a coil 46. Inside the stator 49, a shaft 5s is rotatably inserted.

The disc-type plate 50 is fixed to a lower end part of the supporting member 5. Between the plate 50 and the armature 51, the plate spring 52 is inserted. An end part of the plate spring 52 is fixed to the plate 50, and the other end part thereof is fixed to the armature 51.

Further, the suctioned state of the armature 51 due to the permanent magnet 47 is controlled by switching conduction to the coil 46 of the stator 49. When the coil 46 of the stator 49 is not conducted, the armature 51 is maintained in a fixed state in which the armature 51 is suctioned to the stator 49 due to magnetic force of the permanent magnet 47. At this time, the shaft 5 is maintained fixed to the tip end part 45 of the horizontal arm part 3a. Therefore, the shaft 5s of at the lower end part of the supporting member 5 is maintained to be not rotatable.

When the coil 46 of the stator 49 is switched to a conducted state, the magnetic force of the permanent magnet 47 is lost, and the spring force of the plate spring 52 separates the armature 51 away from the stator 49, as shown in FIG. 5. Therefore, switching is achieved into a state in which the shaft 38 is released from being fixed to the rear link end part 17. Therefore, the shaft 5s of the supporting member 5 is switched to a state of being rotatable.

A second joint rotation part 21k and a second lock part 21 have the same structures as the first joint rotation part 20k and the first lock part 20, and will be therefore omitted from descriptions.

Further, the supporting apparatus 2 for medical device, according to the present embodiment, is provided with a brake switch 19 equivalent to an input part which instructs switching between a fixed state and a fixing release state for the lock part 13, first lock part 20, and second lock part 21. The brake switch 19 is provided on an operation part 10 of the flexible endoscope 11. Here, the brake system of the brake switch 19 exemplifies an alternate method.

FIG. 6 shows a separate state of the drive part 9 of the flexible endoscope 11 and the connection part of the insertion part 8. A transparent photo interrupter 23 as a detector which detects attachment/detachment of the flexible endoscope 11 is attached to the connection part of the drive part 9 to the insertion part 8. A detection target 2 for the photo interrupter 23 is attached to the insertion part 8.

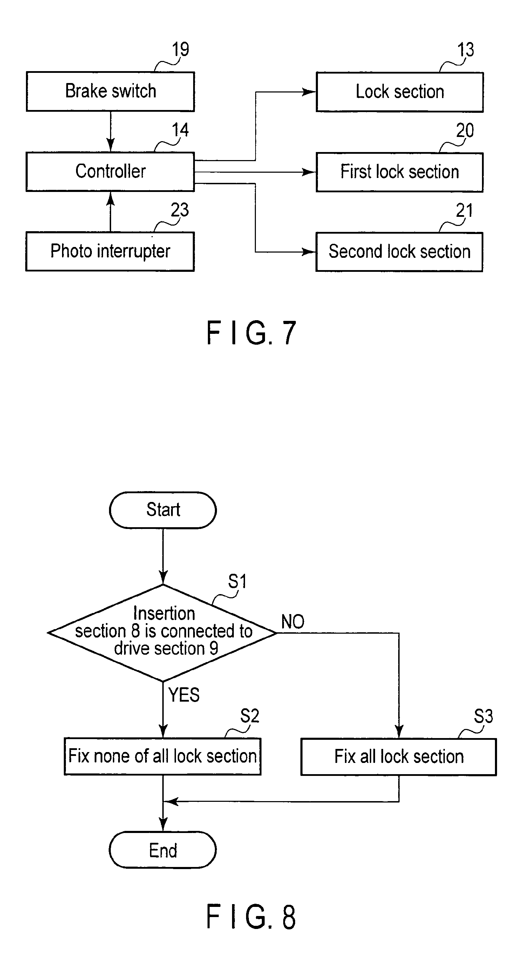

A controller (control unit) 14 is provided on the base plate 1a of the stand unit 1. As shown in FIG. 7, the controller 14 is connected to the brake switch 19, photo interrupter 23, lock part 13, first lock part 20, and each of the electromagnetic brakes 31 and 48 of the second lock part 21. Further, when the photo interrupter 23 detects a state of the insertion part 8 detached from the drive part 9 of the flexible endoscope 11, a control signal to switch the lock part 13, first lock part 20, and each of the electromagnetic brakes 31 and 48 of the second lock part 21 is output, based on a detection signal from the photo interrupter 23. The first joint 4k1 of the link mechanism part 4, first joint rotation part 20k, and second joint rotation part 21k are fixed by the lock part 13, first lock part 20, and the electromagnetic brakes 31 and 48 of the second lock part 21.

Next, operations of the configuration of the aforementioned embodiment will be described referring to a flowchart shown in FIG. 8.

During use of the supporting apparatus 2 for medical device, according to the present embodiment, operations of the lock part 13, first lock part 20, and the electromagnetic brakes 31 and 48 of the second lock part 21 of the supporting apparatus 2 for medical device are controlled.

At first, the supporting apparatus 2 for medical device is powered on to start operating, and the controller 14 then determines whether the insertion part 8 is connected to the drive part 9 or not, by information sent from the transparent photo interrupter 23 to the controller 14 (Step S1). If the insertion part 8 is connected to the drive part 9 (YES) according to the determination, operation ends without fixing the lock part 13, first lock part 20, or electromagnetic brakes 31 and 48 of the second lock part 21 of the supporting apparatus 2 for medical device (Step S2).

Otherwise in Step S1, if the insertion part 8 is determined to be not connected to the drive part 9 (the insertion part 8 is detached from the drive part 9: NO), the lock part 13, first lock part 20, and electromagnetic brakes 31 and 48 of the second lock part 21 of the supporting apparatus 2 for medical device are fixed, and operation stops (Step S3).

Further, if the insertion part 8 is connected to the drive part 9 according to the determination made in the foregoing Step S1, operations of the lock part 13, first lock part 20, and electromagnetic brakes 31 and 48 of the second lock part 21 of the supporting apparatus 2 for medical device are controlled by operating the brake switch 19. When the brake switch 19 is not operated, the lock part 13, first lock part 20, and coils 36 and 46 of the electromagnetic brakes 31 and 48 of the second lock part 21 are maintained not conducted.

In this case, as shown in FIG. 3, the armature 32 is maintained in a fixed state of being suctioned to the stator 37 owing to the magnetic force of the permanent magnet 35 of the lock part 13. At this time, the shaft 38 is maintained fixed to the rear link end part 17. Therefore, the shaft 38 in the upper plate 15 is maintained to be not rotatable, and is thereby maintained in a fixed state of fixing movement of the link mechanism part 4.

Also at this time, the armature 51 is maintained in a fixed state of being suctioned to the stator 49 by the magnetic force of the permanent magnet 47 of the second lock part 21. Therefore, the first joint rotation part 20k and second joint rotation part 21k are maintained in a fixed state of fixing movements thereof.

When the brake switch 19 is pressed once, the coils 36 and 46 of the electromagnetic brakes 31 and 48 of the lock part 13, first lock part 20, and second lock part 21 are conducted. In this case, suction force of the permanent magnet 35 of the lock part 13 of the supporting apparatus 2 for medical device is lost, fixing of the armature 32 and stator 37 is released. Therefore, the upper plate 15 and rear link end part 17 are not fixed. Therefore, the link mechanism part 4 is switched to a state of being movable in up and down directions.

At this time, suction force of the permanent magnet 47 at each of the first lock part 20 and second lock part 21 is lost, and therefore, fixing of the armature 51 and stator 49 is released. Accordingly, the state switches to a fixing release state of releasing fixing of the first joint rotation part 20k and second joint rotation part 21k. Therefore, the link mechanism part 4 can rotate horizontally in relation to the tip end part 45 of the horizontal arm part 3a of the post 3. Similarly, the supporting part 6 can be horizontally pivoted in relation to the tip-link end part 18.

Further, when the brake switch 19 is pressed twice, the brake switch 19 then returns to an unpressed state.

According to the present embodiment as described above, the following effects are performed.

The link mechanism part 4 of the endoscope supporting apparatus 1 according to the present embodiment maintains a balance at an arbitrary position in up and down directions owing to reaction force of the compression spring 12 of the balance mechanism 201 attached to the link mechanism part 4 configured as a parallelogram link by the upper plate 15, lower plate 16, rear link end part 17, and tip-link end part 18.

Further, when the insertion part 8 is detached from the drive part 9, information thereof is sent from the transparent photo interrupter 23 to the controller 14, and each of the electromagnetic brakes 31 and 48 of the lock part 13, first lock part 20, and second lock part 21 of the supporting apparatus 2 for medical device are fixed. Therefore, a tip end of the link mechanism part 4 of the supporting apparatus 2 for medical device does not jump up but stays at their positions.

Accordingly, replacement work for the flexible endoscope 11 adequate for a surgical method during an inspection (surgical operation), and replacement work for the flexible endoscope 11 for disinfection (sterilization) before and after a surgical operation are easy. Further, when the insertion part 8 is detached from the drive part 9, operation for preventing jumping of the tip end of the link mechanism part 4 of the supporting apparatus 2 for medical device is not required. Therefore, there is no new load of work to replace a medical device such as the insertion part 8 of the flexible endoscope 11.

Accordingly, the supporting apparatus for medical device, according to the present embodiment, can maintain a balance of the medical device at an arbitrary position, and can prevent jumping of the tip end of the supporting apparatus for medical device even when the medical device is detached.

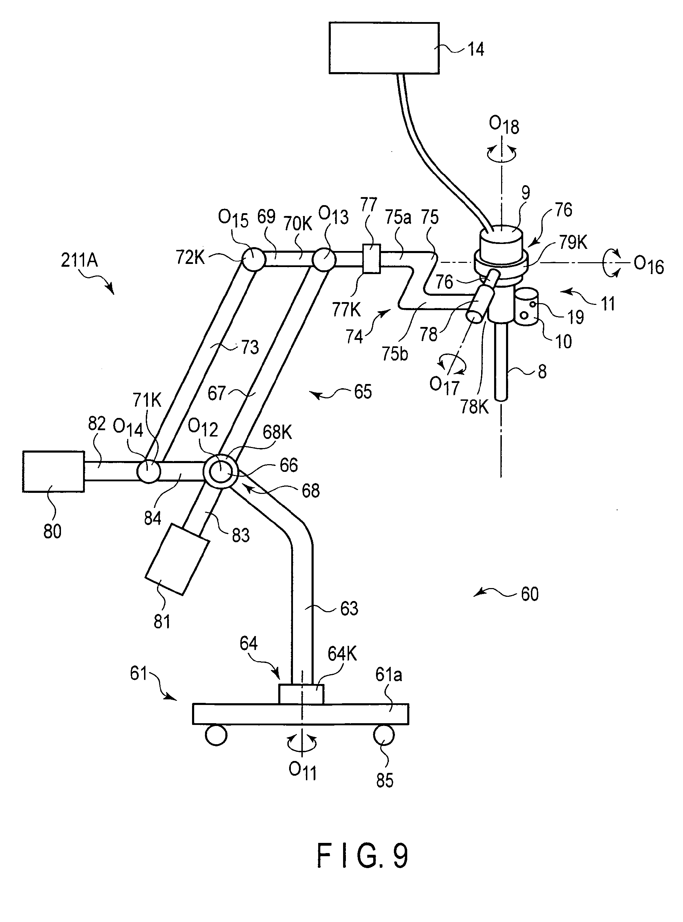

Further, the present embodiment exemplifies, as the supporting apparatus 2 for medical device, the supporting apparatus for medical device comprising the vertical balance mechanism which maintains a balance in the up and down directions owing to reaction force of the compression spring 12. However, the same effects are obtained even with a supporting apparatus for medical device which maintains a three-dimensional balance owing to the principle of balance as shown in FIG. 9. Still further, the present embodiment, exemplifies a flexible endoscope as the medical device. However, the same effects can be obtained even in application to a microscope, a hard mirror, or a treatment instrument as the medical device.

### Second Embodiment

The second embodiment of the invention will be described with reference to FIG. 9.

FIG. 9 shows an entire configuration of a supporting apparatus 60 for medical device, which maintains a three-dimensional balance by the principle of balance.

FIG. 9 shows a stand unit 61 which supports a supporting apparatus 60 for medical device. To the stand unit 61, a plurality of casters 85 to move are attached below a base plate 61a as a base. On the base plate 61a, a post 63 is provided to stand in a perpendicular direction.

At a lower end part of the post 63, there are provided a joint rotation part 64k connected to the base plate 61a, to be pivotal about a perpendicular rotation axis O11, and a first lock part 64 which can switch between a fixed state of fixing movement of the joint rotation part 64k and a fixing release state of releasing fixing of the joint rotation part 64k.

At an upper end part of the post 63, a supporting part 211A for a flexible endoscope 11 is provided. The supporting part 211A comprises a link mechanism part (arm mechanism) 65 made of a parallelogram link which supports the flexible endoscope 11 to be movable. A support member 66 of the link mechanism part 65 is supported to be pivotal by a first joint part 68k about a second rotation axis O12 at right angles to the rotation axis O11 of the post 63.

Further, the support member 66 is provided with a second lock part 68 capable of switching between a fixed state of fixing movement of the first joint part 68k and a fixing release state of releasing fixing of the second joint rotation part 70k.

The link mechanism part 65 comprises four links (a swing arm 67, a connection rod 73, an upper rod 69, and a lower rod 84) and four joint parts (first to fourth joint parts 68k, 70k, 71k, and 72k). The upper rod 69 and lower rod 84 are provided to be parallel to a horizontal direction. The swing arm 67 and connection rod 73 are extended in a longitudinal direction and arranged in parallel.

A lower end part of the swing arm 67 is connected to be pivotal about the second rotation axis O12 by the first joint part 68k at an intersection with an end part of the lower rod 84. An upper end part of the swing arm 67 is connected to be pivotal about a third rotation axis O13 by the second joint rotation part 70k at an intersection with an end part of the upper rod 69. The other end part of the lower rod 84 is connected to be pivotal about a fourth rotation axis O14 by the third joint rotation part 71k at an intersection with an end part of the lower rod 84. An upper end part of the connection rod 73 is connected to be pivotal about a fifth rotation axis O15 by the fourth joint rotation part 72k at an intersection with the other end part of the upper rod 69.

The end part of the upper rod 69 on a side opposite to the fourth joint part 72k is extended to outside through the second joint rotation part 70k, and the supporting part 74 for the flexible endoscope 11 is connected to the extending part. The supporting part 74 is provided with an L-shaped arm 75 and a connection member 76. An L-shape forming part 75a of the L-shaped arm 75 is connected to an extended part of the upper rod 69, to be pivotal about a rotation axis O16 as a center axis of the upper rod 69, through a joint rotation part 77k. Further, the joint rotation part 77k is provided with a third lock part 77 capable of switching between a fixed state of fixing movement of the joint rotation part 77k and a fixing release state of releasing fixing of the joint rotation part 77k.

To a tip end part of the other L-shape forming part 75b of the L-shaped arm 75, a tip end part of the connection member 76 is connected to be pivotal about a rotation axis O17 perpendicular to a rotation axis O16, through the joint rotation part 78k. Further, the joint rotation part 78k is provided with a fourth lock part 78 capable of switching between a fixed state of fixing movement of the joint rotation part 78k and a fixing release state of releasing fixing of the joint rotation part 78k.

Further, a drive part 9 is connected to a tip end part of the connection member 76, to be pivotal about the rotation axis O18. The joint rotation part 79k is provided with a fifth lock part 79. The fifth lock part 79 switches to either a fixed state of fixing movement of the joint rotation part 79k or a release state of releasing fixing of the joint rotation part 79k.

In the fifth lock part 79, the drive part 9 for the flexible endoscope 11 is inserted in an unillustrated insertion hole, and the drive part 9 is pivotal about a rotation axis O18. An insertion part 8 is connected to the drive part 9, and an operation part 10 is connected to the insertion part 8.

The rotation axis O18 is arranged to be perpendicular to the rotation axis O17, and the rotation axes O16, O17, and O18 cross each other at intersections. The link mechanism part 65 made of a parallelogram link is provided with two counter weights (a first counter weight 80 and a second counter weight 81), balanced with the supporting part 74 connected to the flexible endoscope 11.

Here, the first counter weight 80 is attached to be movable along a shaft 82 of the lower rod 84. Further, the second counter weight 81 is attached to be coaxial with the swing arm 67 attached to the support member 66 and to be movable along a shaft 83 provided in an opposite side to the swing arm 67. The supporting part 211A for the flexible endoscope 11 has the configuration as described above.

The first lock part 64 provided on the rotation axis O11 fixes/releases rotation of the post 63 in relation to the base plate 61a of the stand unit 61. The second lock part 68 provided on the rotation axis O12 fixes/releases rotation of the swing arm 67 in relation to the post 63. The third lock part 77 provided on the rotation axis O16 fixes/releases rotation of the supporting part 74 in relation to the upper rod 69. The fourth lock part 78 provided on the rotation axis O17 fixes/releases rotation of the connection member 76 in relation to the L-shape forming part 75b. The lock part 79 provided on the rotation axis O18 fixes/releases rotation of the drive part 9 by an unillustrated solenoid. The flexible endoscope 11 in the present embodiment has the same structure as the first embodiment (see FIG. 6), and the lock parts each have the same structure as shown in FIG. 5. Therefore, detailed descriptions thereof will be omitted.

Next, operations of the configuration of the present embodiment will be described.

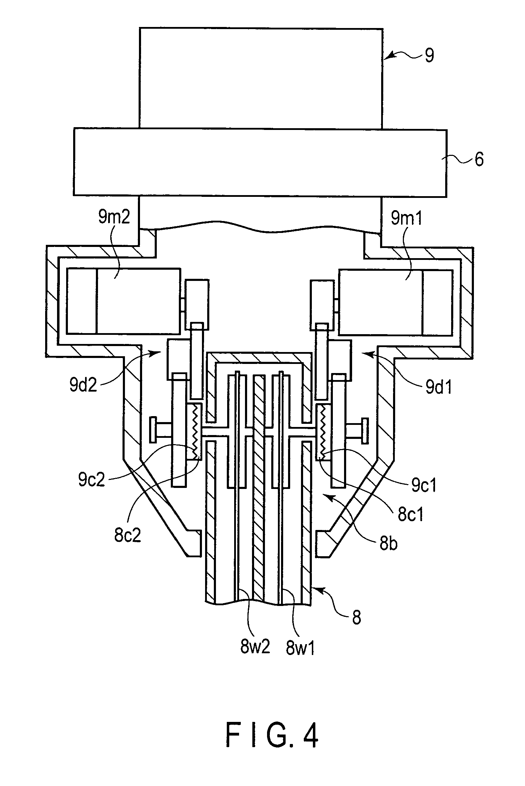

Operations of the first lock part 64, second lock part 68, third lock part 77, fourth lock part 78, and fifth lock part 79 are the same as described previously with reference to FIG. 4. Specifically, when the brake switch 19 is pressed once, none of the joint rotation part 64k between the post 63 and the base plate 61a of the stand unit 61, the first joint part 68k between the swing arm 67 and the post 63, the joint rotation part 77k between the supporting part 74 and the upper rod 69, the joint rotation part 78k between the connection member 76 and the L-shape forming part 75b, and the joint rotation part 79k between the drive part 9 and the lock part 79 are fixed. Therefore, the flexible endoscope 11 is movable.

When the brake switch 19 is not pressed, the first lock part 64 of the joint rotation part 64k, the second lock part 68 of the first joint part 68k, the third lock part 77 of the joint rotation part 77k, the fourth lock part 78 of the joint rotation part 78k are fixed. Therefore, the flexible endoscope 11 is not movable. Further, when the brake switch 19 is pressed twice, the brake switch 19 then returns to an unpressed state.

Regardless of whether the brake switch 19 is pressed or not, when the insertion part 8 is detached from the drive part 9, information thereof is sent from the transparent photo interrupter 23 to the controller 14. The controller 14 then fixes the first lock part 64, second lock part 68, third lock part 77, fourth lock part 78, and fifth lock part 79. Therefore, a tip end of the link mechanism part 65 of the supporting apparatus 60 for medical device stays at the position.

Further in the present embodiment, when the brake switch 19 is pressed one time, the first lock part 64, second lock part 68, third lock part 77, fourth lock part 78, and fifth lock part 79 are fixed. The link mechanism part 65 stays at the position not only in up, down, left, and right directions but also in inclined directions, in addition to the effects of the first embodiment described previously. Accordingly, there is no need for work of manually fixing the flexible endoscope 11 so that the slanting position of the flexible endoscope 11 may not shift before replacement work of the flexible endoscope 11. Therefore, compared with the first embodiment, load of the replacement work for the flexible endoscope 11 is more reduced.

### Third Embodiment

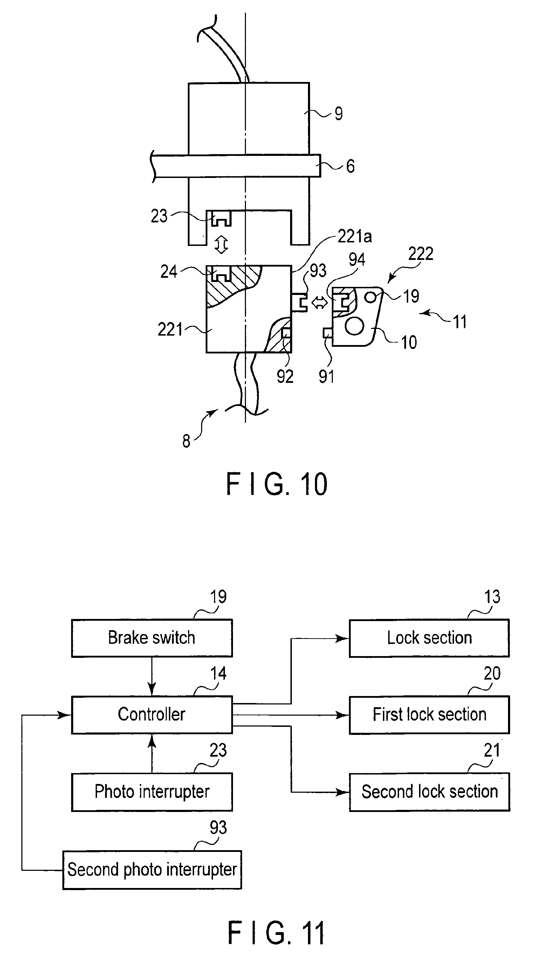

FIGS. 10 to 12 show the third embodiment of the invention. The present embodiment is a modification of the supporting apparatus 2 for medical device according to the first embodiment (see FIGS. 1 to 8). In the present embodiment, only the configuration of a flexible endoscope 11 as a medical device and a control method for fixing/releasing lock parts 13, 20, and 21 of joint rotation parts are different from the first embodiment. Descriptions to the other parts (common to the first embodiment) will be omitted.

FIG. 10 is a cross-sectional view of a main part of the flexible endoscope 11 as a medical device. The flexible endoscope 11 of the present embodiment is configured in a manner that an operation unit (input part) 222 is detachably connected to a base end part 221 of the insertion part 8. The base end part 221 of the insertion part 8 is provided with an operation-part attach/detach part 221a. The operation-part attach/detach part 221a is equipped with a transparent second photo interrupter 93 as a second detector which detects detachment/attachment of the operation unit 222, and is provided with a connection part 92 as a second connection part.

In the operation unit 222, a detection target 94 is attached to a connection part to the operation-part attach/detach part 221a, and is provided with a protruding connection pin 91. When the operation unit 222 is connected to the operation-part attach/detach part 221a, the detection target 94 of the operation unit 222 is detachably engaged with the second photo interrupter 93 of the operation-part detachable part 221a. At this time, the connection pin 91 of the operation unit 222 is detachably engaged in a connection part 92 of the operation-part detachable part 221a.

As shown in FIG. 11, a controller 14 of the supporting apparatus 2 for medical device is connected to a brake switch 19, a photo interrupter 23, a lock part 13, a first lock part 20, and each of electromagnetic brakes 31 and 48 of the second lock part 20, and is also connected to the second photo interrupter 93.

Further, when the insertion part 8 is detected to be detached from the drive part 9 of the flexible endoscope 11 by the photo interrupter 23, a detection signal is output from the photo interrupter 23 to the controller 14. Based on the detection signal, the controller 14 outputs a control signal to switch the lock part 13, the first lock part 20, and the electromagnetic brakes 31 and 48 of the second lock part 21 each to a fixed state. Further, the controller 14 fixes a first joint 4k1, a first joint rotation part 20k, and a second joint rotation part 21k of the link mechanism part 4 by the lock part 13, the first lock part 20, and each of the electromagnetic brakes 31 and 48 of the second lock part 21.

The second photo interrupter 93 detects a state in which the operation unit 222 is detached from the operation-part attach/detach part 221a of the base end part 221 of the insertion part 8. When this detached state is detected, the controller 14 outputs a control signal to switch the lock part 13, the first lock part 20, and the electromagnetic brakes 31 and 48 of the second lock part 21 each to a fixed state, based on a detection signal from the second photo interrupter 93. According to the control signal, the first joint 4k1, first joint rotation part 20k, and second joint rotation part 21k of the link mechanism part 4 are fixed by the lock part 13, first lock part 20, and each of the electromagnetic brakes 31 and 48 of the second lock part 21.

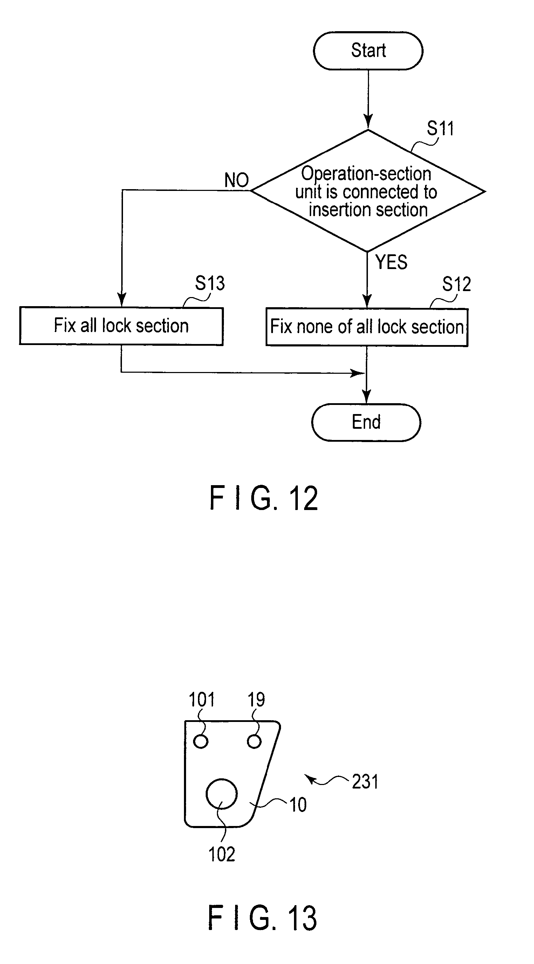

Next, operation of the supporting apparatus 2 for medical device according to the present embodiment will be described referring to the flowchart in FIG. 12.

During use of the supporting apparatus for medical device of the present embodiment, operations of the lock part 13, the first lock part 20, and the electromagnetic brakes 31 and 48 of the second lock part 21 of the supporting apparatus 2 for medical device are controlled.

At first, the supporting apparatus 2 for medical device is powered on to start operating, and the controller 14 then determines whether an operation-part unit 222 is connected to the operation-part attach/detach part 221a of the base end part 221 of the insertion part 8 or not, by information sent from the second photo interrupter 93 to the controller 14 (Step S11). When the operation unit 222 is connected to the operation-part attach/detach part 221a at the base end part 221 of the insertion part 8 (YES), a series of operations end without fixing the lock part 13, first lock part 20, or electromagnetic brakes 31 and 48 of the second lock part 21 of the supporting apparatus 2 for medical device (Step S22).

Otherwise, if the operation unit 222 is detected to be not connected to the operation-part detachable part 221a at the base end part 221 of the insertion part 8 (the operation unit 222 is detached from the operation-part attach/detach part 221a: NO), the lock part 13, first lock part 20, and electromagnetic brakes 31 and 48 of the second lock part 21 of the supporting apparatus 2 for medical device are fixed, and a series of operations stop (Step S3).

In the present embodiment, the following effects are obtained in addition to the effects of the first embodiment.

Regardless of whether the brake switch 19 is pressed or not, when the operation unit 222 is detached from the operation-part attach/detach part 221a at the base end part 221 of the insertion part 8, detachment information is sent from the second photo interrupter 93 to the controller 14. The controller 14 fixes the lock part 13, first lock part 20, and electromagnetic brakes 31 and 48 of the second lock part 21 of the supporting apparatus 2 for medical device, and therefore, the tip end of the second link mechanism part 4 of the supporting apparatus 2 for medical device does not jump up but stays at the position.

Therefore, an inspector (operator) can operate the flexible endoscope 11 with the lock parts 13, 20, and 21 fixed, without limitations to the movable range of the link mechanism part 4 of the supporting apparatus 2 for medical device. Therefore, operationality of the flexible endoscope 11 improves. Since there is no need for work of fixing a tip end of the link mechanism part 4 of the supporting apparatus 2 for medical device, no new work load is applied to replace the flexible endoscope 11.

Further, the present embodiment exemplifies, as the supporting apparatus for medical device, the supporting apparatus for medical device comprising a vertical balance mechanism which maintains a balance in up and down directions owing to reaction force of the compression spring 12 shown in FIG. 1. However, the same effects are obtained even with the supporting apparatus for medical device which maintains a three-dimensional balance owing to principles of balance as shown in FIG. 9.

### Fourth Embodiment

FIGS. 13 to 15 show the first embodiment of the invention. The present embodiment is a modification of the supporting apparatus 2 for medical device, according to the first embodiment (see FIGS. 1 to 8). In the present embodiment, only the control method for fixing/releasing lock parts 13, 20, and 21 of joint rotation parts are different from the first embodiment. Descriptions to the other parts (common to the first embodiment) will be therefore omitted.

FIG. 13 shows a schematic configuration of an operation part 231 of a flexible endoscope 11. The operation part 231 of the flexible endoscope 11 according to the present embodiment is provided with a brake switch 19, a fixing-part change switch 101, and a trackball 102 for bending operation. The trackball 102 is an input device to input an instruction about bending operation of a bending part 8a at a tip end of the insertion part 8.

The fixing portion switch 101 corresponds to a selector for selecting a method for fixing all the lock parts 13, 20, and 21 and a method for fixing only the lock part 13 to move the tip end of the link mechanism part 4 of the supporting apparatus 2 for medical device in up and down directions.

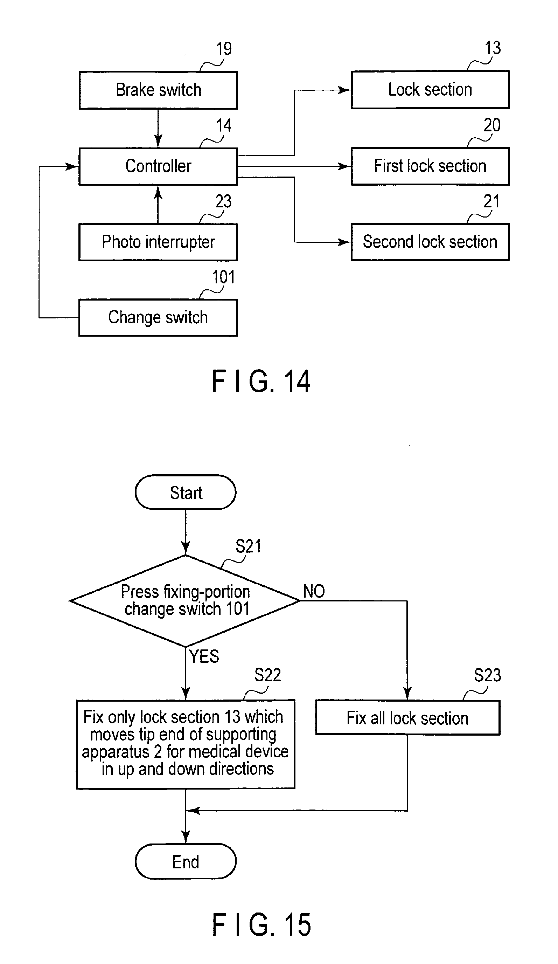

As shown in FIG. 14, a controller 14 of the supporting apparatus 2 for medical device in the present embodiment is connected to a brake switch 19, a photo interrupter 23, a lock part 13, a first lock part 20, and each of electromagnetic brakes 31 and 48 of the second lock part 20, and is newly connected to the fixing-part change switch 101.

During operation of the fixing portion switch 101, a control method of fixing/releasing only the lock part 13 in which the tip end of the link mechanism part 4 of the supporting apparatus 2 for medical device is moved in the up and down directions is set when the fixing-part change switch 101 is pressed once. Further, when the fixing-part change switch 101 is not pressed, a control method of fixing/releasing all the lock parts 13, 20, and 21 is set. Further, when the fixing portion switch 101 is pressed twice, the fixing portion switch 101 then returns to an unpressed state.

Next, operations of the supporting apparatus 2 for medical device of the present embodiment will be described.

The supporting apparatus 2 for medical device is controlled in accordance with the flowchart shown in FIG. 15. Specifically, when the supporting apparatus 2 for medical device is powered on to start operating, the controller 14 determines whether the fixing-part change switch 101 has been pressed or not, by information sent from the fixing-part change switch 101 to the controller 14 (Step S21). In this determination, if the fixing-part change switch 101 is pressed once (YES), a control method of fixing/releasing only the lock part 13 to move the tip end of the link mechanism part 4 of the supporting apparatus 2 for medical device in the up and down directions is set (Step S22).

Further, if the fixing-part change switch 101 is determined to have not been pressed by the determination in step S21 (NO), a control method of fixing/releasing all the lock parts 13, 20, and 21 is set (Step S23). Further, if the fixing-part change switch 101 is pressed twice, the fixing-part change switch 101 then returns to an unpressed state.

Hence, according to the configuration of the present embodiment, either the control method of fixing all the lock parts 13, 20, and 21 or the control method of fixing only the tip end of the supporting apparatus 2 for medical device in the up and down directions can be selected. Therefore, for example, when the insertion part 8 is detached from the drive part 9 after an inspection (surgical operation), the method of fixing only the lock part 13 which moves the tip end of the link mechanism part 4 of the supporting apparatus 2 for medical device in the up and down directions is selected by operation of the fixing portion switch 101. In this manner, the other lock parts 20 and 21 are maintained released from the fixed state, the insertion part 8 can be moved to above a bedside cleaning vessel so that the insertion part 8 can be cleaned immediately.

When the insertion part 8 is attached to the drive part 9 before an inspection (surgical operation), the method of fixing all the lock parts 13, 20, and 21 is selected by operation of the fixing portion switch 101. In this manner, to facilitate attachment work, the work is carried out in a space below the drive part 9 where no obstacle exists. Efficiency of the replacement work before or after an inspection (surgical operation).

Further, the present embodiment exemplifies, as the supporting apparatus 2 for medical device, a supporting apparatus for medical device, comprising an up/down-direction balance mechanism which maintains a balance in the up and down directions owing to reaction force of a compression spring 12. However, the same effects are obtained even with a supporting apparatus for medical device, which maintains a three-dimensional balance owing to the principle of balance.

### Fifth Embodiment

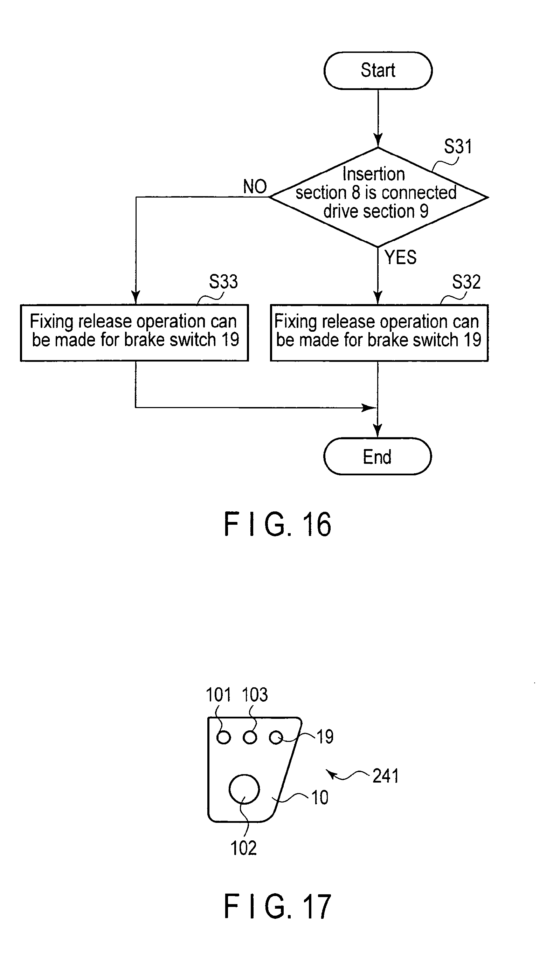

The fifth embodiment of the invention will be described with reference to a flowchart shown in FIG. 16.

The present embodiment is a modification of the supporting apparatus 2 for medical device, according to the first embodiment (see FIGS. 1 to 8). The present embodiment modifies control of a controller of the supporting apparatus 2 for medical device.

Specifically, when the supporting apparatus for medical device is powered on to start operating, the controller 14 determines whether an insertion part 8 is connected to a drive part 9 or not, by information sent from a transparent photo interrupter 23 to the controller 14 (Step S31). According to this determination, if the insertion part 8 is connected to the drive part 9 (YES), operation ends in a state of allowing operation of fixing/releasing a brake switch 19 (Step S32).

Otherwise, according to the determination in Step S31, if the insertion part 8 is not connected to the drive part 9 (the insertion part 8 is detached from the drive part 9: NO), operation ends in a state of not allowing operation of fixing/releasing the brake switch 19 (Step S33).

Hence, according to the present embodiment, the brake switch 19 cannot be operated to be fixed/released when the insertion part 8 is detached from the drive part 9. Accordingly, even when the brake switch 19 is pressed by a mistake, the tip end of the link mechanism part 4 of the supporting apparatus 2 for medical device does not jump up. Therefore, Replacement work for the flexible endoscope 11 is easy.

### Sixth Embodiment

FIGS. 17 to 19 show the sixth embodiment of the invention. The present embodiment is a modification of the supporting apparatus 2 for medical device according to the first embodiment (see FIGS. 1 to 8). An operation part 241 of the flexible endoscope 11 according to the present embodiment is provided with a brake switch 19, a fixing-portion change switch 101, a trackball 102 for bending operation, and an input-part change switch 103.

The input-part change switch 103 corresponds to a selector to select an alternate method in which fixing and release of the joint rotation parts switch over each time the brake switch 19 is pressed, and a momentary method in which fixing of the joint rotation parts is released only while the brake switch 19 is pressed. Selection concerning an input part can be achieved even by double clicking the fixing-portion change switch 101.

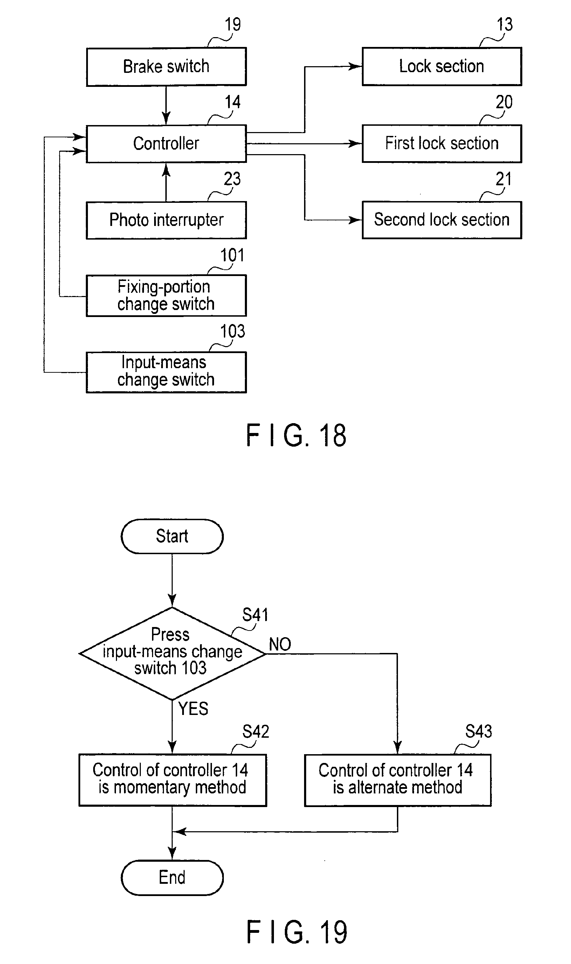

As shown in FIG. 18, a controller 14 of the supporting apparatus 2 for medical device in the present embodiment is connected to the brake switch 19, a photo interrupter 23, a lock part 13, a first lock part 20, each of electromagnetic brakes 31 and 48 of a second lock part 20, and the fixing-portion change switch 101, and is newly connected to the fixing-portion change switch 103.

Further, if the input-part change switch 103 is pressed once during operation of the fixing-portion change switch 101, control according to the momentary method is set, and operation then ends. If the input-part change switch 103 is not pressed, control according to the alternate method is set, and operation then ends. Otherwise, if the input-part change switch 103 is pressed twice, the input-part change switch 103 then returns to an unpressed state.

Next, operation of the supporting apparatus 2 for medical device according to the present embodiment will be described referring to a flowchart shown in FIG. 19.

At first, the supporting apparatus 2 for medical device is powered on to start operating, and the controller 14 then determines whether the input-part change switch 103 has been pressed or not, by information sent from the input-part change switch 103 to the controller 14 (Step S41). According to this determination, if the input-part change switch 103 is pressed once (YES), control according to the momentary method is set, and a series of operations then end.

Otherwise, according to the determination in Step S41, if the input-part change switch 103 is determined to have not been pressed (NO), the control according to the alternate method is set (Step S43), and a series of operations end. Further, if the input-part change switch 103 is pressed twice, the input-part change switch 103 then returns to an unpressed state.

Hence, according to the present embodiment, an input part for fixing/releasing of the joint rotation parts can be selected between the alternate method and the momentary method by the input-part change switch 103. Accordingly, an adequate input part can be selected for an inspection (surgical operation), and operationality of the medical device therefore improves.

The invention is not limited to the embodiments described above but can be variously modified and practiced without deviating from the subject matters of the invention as defined in claim 1.

Next, the embodiments described above have technical features, operations, and effects as described below.

According to one aspect of the invention, a supporting apparatus for medical device comprises: a supporting means comprising a stand unit set on a mount part including a floor, a ceiling, and a trolley, and a balance mechanism which is connected to the stand unit, comprises an arm mechanism comprising a plurality of joint parts, movably and tiltably supports a medical device supported by the arm mechanism, and balances a weight of the medical device and the arm mechanism; a brake means which can switch a fixed state of fixing at least one of the joint parts of the supporting means and a fixing release state of releasing the fixing of the at least one of the joint parts; and an input means which gives an instruction on switching between the fixed state and the fixing release state of the brake means, wherein the supporting means comprises a detection means which detects attachment/detachment of the medical device, and a control means is provided which outputs a control signal to switch the brake means to the fixed state, based on a detection signal from the detection means, and fixes the joint parts by the brake means, when a state of the medical device detached from the supporting means is detected by the detection means.

In the configuration described above, at the time of attaching/detaching the medical device to/from the detection means, the state of the medical device detached from the supporting means is detected by the detection means. In this case, based on a detection signal from the detection means, a control signal which switches the brake means to the fixed state is output by the control means, and the joint parts are fixed by the brake means. In this manner, when the medical device is detached from the supporting means at an arbitrary position, the tip end of the arm mechanism of the supporting means stays at the spot. Therefore, replacement work for the medical device is facilitated.

Preferably, the balance mechanism is either a counter weight mechanism which maintains a three-dimensional balance by at least a counter weight or a spring mechanism which maintains a balance in up and down directions by reaction force of a spring.

Further, in the above configuration, the medical device can be movably and tiltably maintained with the weight of the medical device and the arm mechanism balanced by either the counter weight mechanism which maintains a three-dimensional balance by at least a counter weight or the spring mechanism which maintains a balance in up and down directions by reaction force of a spring.

Preferably, the medical device comprises a second detection means which detects attachment/detachment of the input means, and the control means outputs a control signal to switch the brake means to the fixed state, based on a detection signal from the second detection means, and fixes the joint parts by the brake means, when a state of the input means detached from the medical device is detected by the second detection means.

In the above configuration, attachment/detachment of the input means to/from the medical device is detected by the second detection means. Further, when the state of the input means detached from the medical device is detected by the second detection means, a control signal to switch the brake means to the fixed state is output, based on a detection signal from the second detection means, and the joint parts are fixed by the brake means. In this manner, when the input means is detached from the medical device, a tip end of the arm mechanism of the supporting means does not jump up but stays at the spot. Therefore, work for fixing the tip end of the arm mechanism of the supporting means is not required, and there is no new work load for replacing the medical device. Replacement work for the medical device can be easily performed.

Preferably, the brake means can be switched to either a fixed state of fixing all the joint parts and a fixing release state of releasing the fixing of the joint parts.

In the above configuration, switching is performed between the fixed state of fixing movement of all the joint parts by the brake means and the fixing release state of releasing fixing of the joint parts. In this manner, the medical device can be positioned to an arbitrary position by one operation, and therefore, positioning of the medical device is facilitated.

Preferably, the brake means comprises a selection means which selects one of a method of fixing all the joint parts and a method of fixing only one of the joint parts that moves the medical device in the up and down directions.

In the above configuration, one of a method of fixing all the joint parts and a method of fixing only one of the joint parts that moves the medical device in the up and down directions is selected by the selection means of the brake means. In this manner, when the insertion part is detached from the drive part after an inspection (surgical operation), detachment work is carried out above a bedside cleaning vessel so that the insertion part can be cleaned immediately. When the insertion part is attached to the drive part before an inspection (surgical operation), attachment work is carried out in a space where no obstacle exists below the drive part so that attachment work is facilitated. Efficiency of replacement work before and after an inspection (surgical operation) improves.

Preferably, the brake means comprises an input lock means which does not allow the input means to release fixing of the joint parts when the medical device is detached from the supporting means.

Further, in the above configuration, when the medical device is detached from the supporting means, fixing of the joint parts cannot be achieved by the input lock means. In this manner, replacement work efficiency is improved before and after an inspection (surgical operation) in which the medical device jumps up even when a brake switch is pressed.

Preferably, the input means comprises an press-button-type operation part, and the brake means comprises an alternate method in which the fixed state of the joint parts and the fixing release state thereof change over each time when the press-button-type operation part is operated, and a momentary method in which the fixing of the joint parts is released only while the operation part of the press-button type is pressed. The operation part further comprises a selection means to make selection from the alternate method and the momentary method.

Further, in the above configuration, an operating state of the brake means is selected from either the alternate method or the momentary method by the selection means of the operation part if necessary. In this manner, when the alternate method is selected, the fixed state and the fixing release state of the joint parts are switched over each time when the press-button-type operation part is pressed. Otherwise, when the momentary method is selected, fixing of the joint parts is released only while the press-button-type operation part is pressed.

Preferably, the medical device is an endoscope.

Still further in the above configuration, at the time of attaching/detaching the endoscope to/from the supporting means, a state of the endoscope detached from the supporting means is detected by the detection means. In this case, based on a detection signal from the detection means, a control signal which switches the brake means to the fixed state is output by the control means, and the joint parts are fixed by the brake means. In this manner, when the endoscope is detached from the supporting means at an arbitrary position, the tip end of the arm mechanism of the supporting means stays at the spot. Therefore, replacement work for the endoscope is facilitated.

Preferably, the endoscope comprises an insertion part which is inserted into a body and can be operated to bend, and a drive part which is connected to a base end part of the insertion part and drives the bending part to operate. The drive part is fixed to the supporting means, and the detection means detects that the insertion part is attached/detached to/from the drive part.

Further in the above configuration, when the drive part of the endoscope is fixed to the supporting means and the insertion part is connected detachably to the drive part, a state of the insertion part detached from the drive part is detected by the detection means. In this case, based on a detection signal from the detection means, a control signal which switches the brake means to the fixed state is output by the control means, and the joint parts are fixed by the brake means. In this manner, when the insertion part of the endoscope is detached from the drive part fixed to the supporting means at an arbitrary position, the tip end of the arm mechanism of the supporting means stays at the spot. Therefore, replacement work for the insertion part of the endoscope is facilitated.

## Claims

1. A supporting apparatus (2) for medical device (11), comprising:
a stand unit (1);
a supporting section (2A) which is connected to the stand unit (1), comprises an arm mechanism (4) including a plurality of joint parts (4K1, 4K2, 4K3, 4K4), and is configured to movably and tiltably support a medical device (11) supported by the arm mechanism (4);
a brake section (13) which is configured to switch between a fixed state of fixing at least one of the joint parts (4K1, 4K2, 4K3, 4K4) of the supporting section (2A) and a fixing release state of releasing the fixing of the at least one of the joint parts (4K1, 4K2, 4K3, 4K4); and
a detector (23) which is comprised by the supporting section (2A) and is configured to detect attachment/detachment of the medical device (11);
wherein the supporting apparatus (2) further comprises a controller (14) configured to output a control signal to the brake section (13); **characterized in that** the controller (14) is configured to output the control signal to switch the brake section (13) to a fixed state, based on a detection signal from the detector (23), and fix the joint parts (4K1, 4K2, 4K3, 4K4) by the brake section (13), when the detector (23) detects that the medical device (11) is detached from the supporting section (2A).

2. The supporting apparatus (2) for medical device (11), according to claim 1, wherein
the supporting section (2A) comprises a balance mechanism (201) configured to balance a weight of the medical device (11) and the arm mechanism (4).

3. The supporting apparatus (2) for medical device (11), according to claim 2, wherein
the balance mechanism (201) is either a counter weight mechanism configured to maintain a three-dimensional balance by at least a counter weight or a spring mechanism configured to maintain a balance in up and down directions by reaction force of a spring.

4. The supporting apparatus (2) for medical device (11), according to claim 2, wherein
the supporting apparatus (2) for medical device (11) comprises an input section (10) configured to give an instruction on switching between the fixed state and the fixing release state of the brake section (13).

5. The supporting apparatus (2) for medical device (11), according to claim 4, wherein
the medical device (11) comprises a second detector (93) configured to detect attachment/detachment of the input section (10), and
the controller (14) is configured to output a control signal to switch the brake section (13) to the fixed state, based on a detection signal from the second detector (93), and fix the joint parts (4K1, 4K2, 4K3, 4K4) by the brake section (13), when a state of the input section (10) detached from the medical device (11) is detected by the second detector (93).

6. The supporting apparatus (2) for medical device (11), according to claim 2, wherein
the brake section (13) is configured to switch to either the fixed state of fixing at least one of all the joint parts (4K1, 4K2, 4K3, 4K4) and the fixing release state of releasing the fixing of the joint parts (4K1, 4K2, 4K3, 4K4).

7. The supporting apparatus (2) for medical device (11), according to claim 6, wherein
the brake section (13) comprises a selector (101) configured to select and implement one of a method of fixing all the joint parts (4K1, 4K2, 4K3, 4K4) and a method of fixing only one of the joint parts (4K1, 4K2, 4K3, 4K4) that moves the medical device (11) in the up and down directions.

8. The supporting apparatus (2) for medical device (11), according to claim 2, wherein
the brake section (13) comprises an input lock section (19) configured to not allow the input section (10) to make a fixing release operation of the joint parts (4K1, 4K2, 4K3, 4K4) when the medical device (11) is detached from the supporting section (2A).

9. The supporting apparatus (2) for medical device (11), according to claim 4, wherein
the input section (10) comprises an operation section (19) of a press-button type,
the brake section (13) is configured to implement an alternate method in which the fixed state of the joint parts (4K1, 4K2, 4K3, 4K4) and the fixing release state change over each time the operation section (19) of the press-button type is operated, and a momentary method in which the fixing of the joint parts (4K1, 4K2, 4K3, 4K4) is released only while the operation part of the press-button type is pressed, and
the operation section (19) comprises a selector (103) configured to make a selection from the alternate method and the momentary method.

10. The supporting apparatus (2) for medical device (11), according to claim 2, wherein
the medical device is an endoscope that comprises an insertion section (8) configured to be inserted into a body cavity and comprising a bending section (8a) configured to be bent, and a drive section (9) connected to a proximal end of the insertion section and configured to drive the bending section (8); and wherein a base end part of the insertion section (8) of the endoscope is detachably connected to the drive section (9).

11. The supporting apparatus (2) for medical device (11), according to claim 10, wherein
the drive section (9) can operate a bending section (8a) of the insertion section (8).

## Patentansprüche

1. Trägergerät (2) für ein medizinisches Gerät (11), das umfasst:
eine Standeinheit (1);
einen Trägerabschnitt (2A), der mit der Standeinheit (1) verbunden ist, der einen Armmechanismus (4) umfasst, welcher eine Mehrzahl von Gelenkteilen (4K1, 4K2, 4K3, 4K4) umfasst, und der dazu eingerichtet ist, ein medizinisches Gerät (11) beweglich und neigbar abzustützen, das durch den Armmechanismus (4) abgestützt wird;
einen Bremsabschnitt (13), der dazu eingerichtet ist, zwischen einem fixierten Zustand, in dem mindestens eines der Gelenkteile (4K1, 4K2, 4K3, 4K4) des Trägerabschnitts (2A) fixiert ist, und einem Fixierungslösezustand, in dem die Fixierung mindestens eines der Gelenkteile (4K1, 4K2, 4K3, 4K4) gelöst ist, umzuschalten; und
einen Detektor (23), der durch den Trägerabschnitt (2A) umfasst ist und der dazu eingerichtet ist, eine Montage/Demontage des medizinischen Geräts (11) zu detektieren;
wobei
das Trägergerät (2) ferner eine Steuerung (14) umfasst, die dazu eingerichtet ist, ein Steuerungssignal an den Bremsabschnitt (13) auszugeben;
**dadurch gekennzeichnet, dass**
die Steuerung (14) dazu eingerichtet ist, das Steuerungssignal basierend auf einem Detektionssignals des Detektors (23) auszugeben, um den Bremsabschnitt (13) in einen fixierten Zustand zu schalten und
die Gelenkteile (4K1, 4K2, 4K3, 4K4) durch den Bremsabschnitt (13) zu fixieren, wenn der Detektor (23) detektiert, dass das medizinische Geräte (11) von dem Trägerabschnitt (2A) gelöst ist.

2. Trägergerät (2) für ein medizinisches Gerät (11) gemäß Anspruch 1, wobei
der Trägerabschnitt (2A) einen Ausgleichsmechanismus (201) umfasst, der dazu eingerichtet ist, ein Gewicht des medizinischen Geräts (11) und des Armmechanismus (4) auszugleichen.

3. Trägergerät (2) für ein medizinisches Gerät (11) gemäß Anspruch 2, wobei
der Ausgleichsmechanismus (201) entweder ein Gegengewichtsmechanismus, der dazu eingerichtet ist, einen dreidimensionalen Ausgleich durch mindestens ein Gegengewicht zu erhalten, oder ein Federmechanismus ist, der dazu eingerichtet ist, einen Ausgleich in auf- und abwärts gerichteten Richtungen durch eine Reaktionskraft einer Feder zu erhalten.

4. Trägergerät (2) für ein medizinisches Gerät (11) gemäß Anspruch 2, wobei das Trägergerät (2) für ein medizinisches Gerät (11) einen Eingabeabschnitt (10) umfasst, der dazu eingerichtet ist, eine Anweisung zum Umschalten zwischen dem fixierten Zustand und dem Fixierungslösezustand des Bremsabschnitts (13) auszugeben.

5. Trägergerät (2) für ein medizinisches Gerät (11) gemäß Anspruch 4, wobei
das medizinische Gerät (11) einen zweiten Detektor (93) umfasst, der dazu eingerichtet ist, eine Montage/Demontage des Eingabeabschnitts (10) zu detektieren und
die Steuerung (14) dazu eingerichtet ist, basierend auf einem Detektionssignal des zweiten Detektors (93) ein Steuersignal auszugeben, um den Bremsabschnitt (13) in den fixierten Zustand zu schalten und
die Gelenkteile (4K1, 4K2, 4K3, 4K4) durch den Bremsabschnitt (13) zu fixieren, wenn ein von dem medizinischen Gerät (11) gelöster Zustand des Eingabeabschnitts (10) durch den zweiten Detektor (93) detektiert wird.

6. Trägergerät (2) für ein medizinisches Gerät (11) gemäß Anspruch 2, wobei
der Bremsabschnitt (13) dazu eingerichtet ist, entweder in den fixierten Zustand, in dem mindestens eines aller Gelenkteile (4K1, 4K2, 4K3, 4K4) fixiert ist, oder in den Fixierungslösezustand, in dem die Fixierung der Gelenkteile (4K1, 4K2, 4K3, 4K4) gelöst ist, zu schalten.

7. Trägergerät (2) für ein medizinisches Gerät (11) gemäß Anspruch 6, wobei
der Bremsabschnitt (13) einen Wahlschalter (101) umfasst, der dazu eingerichtet ist, entweder ein Verfahren zur Fixierung aller Gelenkteile (4K1, 4K2, 4K3, 4K4) oder ein Verfahren zur Fixierung von nur einem der Gelenkteile (4K1, 4K2, 4K3, 4K4), das das medizinische Gerät (11) in den auf- und abwärts gerichteten Richtungen bewegt, auszuwählen und umzusetzen.

8. Trägergerät (2) für ein medizinisches Gerät (11) gemäß Anspruch 2, wobei
der Bremsabschnitt (13) einen Eingabespeerabschnitt (19) umfasst, der dazu eingerichtet ist, zu unterbinden, dass der Eingabeabschnitt (10) eine Fixierungslöseoperation der Gelenkteile (4K1, 4K2, 4K3, 4K4) ausführt, wenn das medizinische Gerät (11) von dem Trägerabschnitt (2A) gelöst ist.

9. Trägergerät (2) für ein medizinisches Gerät (11) gemäß Anspruch 4, wobei
der Eingabeabschnitt (10) einen Betätigungsabschnitt (19) vom Typ eines Druckknopfs umfasst,
der Bremsabschnitt (13) dazu eingerichtet ist, ein alternatives Verfahren, bei dem bei jeder Betätigung des Betätigungsabschnitts (19) vom Typ eines Druckknopfs zwischen dem fixierten Zustand der Gelenkteile (4K1, 4K2, 4K3, 4K4) und dem Fixierungslösezustand gewechselt wird, und ein aktuelles Verfahren umzusetzen, bei dem die Fixierung der Gelenkteile (4K1, 4K2, 4K3, 4K4) nur gelöst wird, während der Betätigungsteil vom Typ eines Druckknopf gedrückt ist, und
der Betätigungsabschnitt (19) einen Wahlschalter (103) umfasst, der dazu eingerichtet ist, eine Auswahl zwischen dem alternativen Verfahren und dem aktuellen Verfahren zu treffen.

10. Trägergerät (2) für ein medizinisches Gerät (11) gemäß Anspruch 2, wobei
das medizinische Gerät ein Endoskop ist, das einen Einführabschnitt (8) umfasst, der dazu eingerichtet ist, in einen Körperhohlraum eingeführt zu werden und einen Biegeabschnitt (8a), der dazu eingerichtet ist, gebogen zu werden und einen Antriebsabschnitt (9) umfasst, der mit einem proximalen Ende des Einführabschnitts verbunden ist und dazu eingerichtet ist, den Biegeabschnitt (8) anzutreiben; und wobei ein Basisendteil des Einführabschnitts (8) des Endoskops mit dem Antriebsabschnitt (9) lösbar verbunden ist.

11. Trägergerät (2) für ein medizinisches Gerät (11) gemäß Anspruch 10, wobei
der Antriebsabschnitt (9) einen Biegeabschnitt (8a) des Einführabschnitts (8) betätigen kann.

## Revendications

1. Appareil de support (2) pour dispositif médical (11), comprenant :
une unité de support (1) ;
une section de support (2A) qui est connectée à l'unité de support (1), comprend un mécanisme de bras (4) comprenant une pluralité de parties de joints (4K1, 4K2, 4K3, 4K4), et est configurée pour supporter de manière mobile et inclinable un dispositif médical (11) supporté par le mécanisme de bras (4) ;
une section de frein (13) qui est configurée pour commuter entre un état fixé consistant à fixer au moins l'une des parties de joints (4K1, 4K2, 4K3, 4K4) de la section de support (2A) et un état de libération de fixation consistant à libérer la fixation de la au moins une des parties de joints (4K1, 4K2, 4K3, 4K4) ; et
un détecteur (23) qui est contenu par la section de support (2A) et est configuré pour détecter la fixation/séparation du dispositif médical (11) ;
dans lequel l'appareil de support (2) comprend en outre un contrôleur (14) configuré pour délivrer en sortie un signal de commande vers la section de frein (13) ;
**caractérisé en ce que** le contrôleur (14) est configuré pour délivrer en sortie le signal de commande pour commuter la section de frein (13) vers un état fixé, sur la base d'un signal de détection provenant du détecteur (23), et fixer les parties de joints (4K1, 4K2, 4K3, 4K4) par la section de frein (13), lorsque le détecteur (23) détecte que le dispositif médical (11) est détaché de la section de support (2A).

2. Appareil de support (2) pour dispositif médical (11) selon la revendication 1, dans lequel
la section de support (2A) comprend un mécanisme d'équilibrage (201) configuré pour équilibrer un poids du dispositif médical (11) et du mécanisme de bras (4).

3. Appareil de support (2) pour dispositif médical (11) selon la revendication 2, dans lequel
le mécanisme d'équilibrage (201) est soit un mécanisme de contrepoids configuré pour maintenir un équilibre en trois dimensions par au moins un contrepoids soit un mécanisme de ressort configuré pour maintenir un équilibre dans les sens haut et bas par la force de réaction d'un ressort.

4. Appareil de support (2) pour dispositif médical (11) selon la revendication 2, dans lequel
l'appareil de support (2) pour dispositif médical (11) comprend une section d'entrée (10) configurée pour délivrer une instruction de commutation entre l'état fixé et l'état de libération de fixation de la section de frein (13).

5. Appareil de support (2) pour dispositif médical (11) selon la revendication 4, dans lequel
le dispositif médical (11) comprend un deuxième détecteur (93) configuré pour détecter la fixation/séparation de la section d'entrée (10), et
le contrôleur (14) est configuré pour délivrer en sortie un signal de commande pour commuter la section de frein (13) vers l'état fixé, sur la base d'un signal de détection provenant du deuxième détecteur (93), et fixer les parties de joints (4K1, 4K2, 4K3, 4K4) par la section de frein (13), lorsqu'un état de la section d'entrée (10) détachée du dispositif médical (11) est détecté par le deuxième détecteur (93).

6. Appareil de support (2) pour dispositif médical (11) selon la revendication 2, dans lequel
la section de frein (13) est configurée pour commuter soit vers l'état fixé consistant à fixer au moins l'une parmi toutes les parties de joints (4K1, 4K2, 4K3, 4K4) soit vers l'état de libération de fixation consistant à libérer la fixation des parties de joints (4K1, 4K2, 4K3, 4K4).

7. Appareil de support (2) pour dispositif médical (11) selon la revendication 6, dans lequel
la section de frein (13) comprend un sélecteur (101) configuré pour sélectionner et mettre en oeuvre l'un parmi un procédé consistant à fixer toutes les parties de joints (4K1, 4K2, 4K3, 4K4) et un procédé consistant à fixer uniquement l'une des parties de joints (4K1, 4K2, 4K3, 4K4) qui déplace le dispositif médical (11) vers le haut et vers le bas.

8. Appareil de support (2) pour dispositif médical (11) selon la revendication 2, dans lequel
la section de frein (13) comprend une section de blocage d'entrée (19) configurée pour ne pas permettre à la section d'entrée (10) d'effectuer une opération de libération de fixation des parties de joints (4K1, 4K2, 4K3, 4K4) lorsque le dispositif médical (11) est détaché de la section de support (2A).

9. Appareil de support (2) pour dispositif médical (11) selon la revendication 4, dans lequel
la section d'entrée (10) comprend une section de commande (19) d'un type bouton-poussoir,
la section de frein (13) est configurée pour mettre en oeuvre un procédé alternatif dans lequel l'état fixé des parties de joints (4K1, 4K2, 4K3, 4K4) et l'état de libération de fixation permutent chaque fois que la section de commande (19) du type bouton-poussoir est actionnée, et un procédé momentané dans lequel la fixation des parties de joints (4K1, 4K2, 4K3, 4K4) est libérée uniquement lorsque la partie de commande du type bouton-poussoir est enfoncée, et
la section de commande (19) comprend un sélecteur (103) configuré pour effectuer une sélection parmi le procédé alternatif et le procédé momentané.

10. Appareil de support (2) pour dispositif médical (11) selon la revendication 2, dans lequel
le dispositif médical est un endoscope qui comprend une section d'insertion (8) configurée pour être insérée dans une cavité de corps et comprenant une section de courbure (8a) configurée pour être courbée, et une section d'entraînement (9) connectée à une extrémité proximale de la section d'insertion et configurée pour entraîner la section de courbure (8a) ; et
dans lequel une partie d'extrémité de base de la section d'insertion (8) de l'endoscope est connectée de manière détachable à la section d'entraînement (9).

11. Appareil de support (2) pour dispositif médical (11) selon la revendication 10, dans lequel
la section d'entraînement (9) peut actionner une section de courbure (8a) de la section d'insertion (8).
